# FASCICULE DE BREVET EUROPEEN

(11) **EP 0 850 414 B1**
(45) Date de publication et mention de la délivrance du brevet: **20.08.2003**
(21) Numéro de dépôt: 97931851.6
(22) Date de dépôt: 03.07.1997
(51) Int. Cl.: G01N 33/36, D02J 1/22

(54) **DISPOSITIF POUR LA FABRICATION D'UN FIL COMPOSITE**
VORRICHTUNG ZUR HERSTELLUNG EINER VERBUNDFASER
DEVICE FOR MANUFACTURING A COMPOSITE YARN

(30) Priorité: 10.07.1996 FR 9608592
(43) Date de publication de la demande: 01.07.1998
(73) Titulaire: Saint-Gobain Vetrotex France, 73000 Chambéry (FR)
(72) Inventeur: LOUBINOUX, Dominique, F-38660 La Terrasse (FR); RICHARD, Daniel, F-73800 Sainte-Hélène-du-Lac (FR)
(74) Mandataire: Breton, Jean-Claude
(86) Numéro de dépôt international: FR9701184
(87) Numéro de publication internationale: WO98001751

(56) Documents cités:
- EP-A- 0 505 274
- EP-A- 0 610 147
- DE-A- 3 617 248
- US-A- 5 422 180

## Description

La présente invention concerne un dispositif de fabrication d'un fil composite formé par l'association d'une multiplicité de filaments continus de verre et de filaments organiques thermoplastiques.

Plus précisément, l'invention concerne un dispositif comprenant au moins une filière alimentée en verre, à partir de laquelle sont étirés une multiplicité de filaments de verre continus, et au moins une tête de filage alimentée en matière organique thermoplastique, à partir de laquelle sont également étirés une multiplicité de filaments organiques continus. Ces filaments organiques sont étirés et projetés au moyen d'un dispositif venturi dans la nappe de filaments de verre en cours d'étirage, l'ensemble desdits filaments étant ensuite rassemblés sous la forme d'un fil composite. Une telle installation est décrite par exemple dans la demande de brevet FR-A-2 698 038.

Dans le cadre de l'invention décrite dans cette demande, les filaments thermoplastiques sont étirés séparément des filaments de verre au moyen de tambours dont la vitesse de rotation est commandée de manière à conférer à la nappe de filaments thermoplastiques une vitesse supérieure à la vitesse d'étirage des filaments de verre. La nappe de filaments thermoplastiques, éventuellement guidée, passe dans un dispositif venturi qui assure l'orientation et la projection de ladite nappe dans la nappe de filaments de verre en cours d'étirage. Le dispositif, qui maintient la vitesse conférée aux filaments thermoplastiques par les tambours d'étirage, assure uniquement la projection desdits filaments dans la nappe des filaments de verre en les maintenant individualisés. La différence ainsi créée entre les vitesses d'étirage des deux catégories de filaments a pour effet de compenser le retrait des filaments thermoplastiques qui intervient avant le bobinage du fil composite. Il est ainsi possible d'obtenir des bobines de fil composite dont l'ensemble des filaments qui le constitue sont de même longueur. L'apparition d'un écart entre les vitesses désirées pour les filaments, même lorsqu'il est faible, peut se traduire par un défaut au niveau du produit obtenu ou par un mauvais fonctionnement de l'installation.

Il en est ainsi lorsqu'un écart se produit entre la vitesse conférée aux filaments thermoplastiques par les tambours d'étirage et la vitesse desdits filaments lorsqu'ils entrent en contact avec les filaments de verre.

La présente invention a pour objet un dispositif de fabrication d'un fil composite dans lequel la vitesse de rotation des tambours et la vitesse des filaments thermoplastiques lorsqu'ils entrent en contact avec les filaments de verre sont automatiquement synchronisés.

La présente invention a pour objet un dispositif de fabrication d'un fil composite dans lequel la synchronisation automatique des vitesses considérées peut être effectuée quelles que soient la nature du thermoplastique employé et la vitesse adoptée pour l'étirage des filaments thermoplastiques.

Ces objectifs sont atteints par un dispositif pour la fabrication d'un fil composite formé par l'association de filaments continus de verre et de filaments continus de matière organique thermoplastique comprenant d'une part au moins une filière, alimentée en verre, dont la face inférieure est munie d'une multiplicité d'orifices à partir desquels sont étirés une multiplicité de filaments de verre, associée à un dispositif enducteur, d'autre part au moins une tête de filage alimentée en matière organique thermoplastique fondue, dont la face inférieure est munie d'une multiplicité d'orifices à partir desquels sont étirés une multiplicité de filaments organiques, ladite tête étant associée à un dispositif d'étirage comprenant au moins deux tambours et à un dispositif venturi projetant les filaments organiques dans la nappe formée par les filaments de verre, et des moyens, communs à la filière et à la tête de filage, permettant l'assemblage et l'étirage du fil composite, par exemple par un dispositif de bobinage tel qu'un bobinoir muni de broches, dans lequel un dispositif optique de détection de la nappe de filaments thermoplastiques défilant dans une zone déterminée est disposé entre le dispositif d'étirage à tambour et le dispositif venturi, ledit dispositif optique étant relié à un circuit commandant la vitesse de rotation dont au moins un des tambours.

Ce dispositif de détection comprend au moins deux détecteurs placés sur un support à des hauteurs différentes qui définissent les limites d'une zone dans laquelle ladite nappe peut se déplacer pendant l'opération d'étirage des filaments thermoplastiques.

Dans ce dispositif de détection, les détecteurs peuvent être disposés d'un seul côté de la nappe de filaments. Chaque détecteur comprend un émetteur et un récepteur, le premier émettant un faisceau lumineux en direction de la nappe, le second recevant au moins une partie de la lumière réfléchie par les filaments rencontrant ledit faisceau.

Les détecteurs peuvent être également constitués d'émetteurs et de récepteurs disposés de part et d'autre de la nappe ; chaque émetteur coopère alors avec un récepteur qui reçoit ou non le faisceau lumineux qu'il émet, selon l'absence ou la présence de filaments dans la zone traversée par ledit faisceau.

Les émetteurs utilisés émettant de préférence, un faisceau lumineux par pulsations régulières. La reconnaissance de ces pulsations par les récepteurs permet de réduire, voire d'éliminer les signaux parasites susceptibles d'être enregistrés par lesdits capteurs en provenance d'autres sources lumineuses que lesdits émetteurs.

La reconnaissance par chaque récepteur de la présence ou de l'absence de filaments dans son champ d'examen est transmise à un amplificateur qui envoie un signal binaire à un automate. Cet automate possède une sortie de commande analogique ou digitale qui est reliée à l'entrée de la consigne de vitesse du dispositif de commande du ou des moteurs assurant la rotation des tambours d'étirage.

Suivant la position plus ou moins tendue de la nappe de filaments entre le dernier tambour d'étirage et le dispositif venturi, les détecteurs transmettent périodiquement à l'automate, via l'amplificateur, une série de signaux binaires. Selon la nature de ces signaux, l'automate commande l'augmentation ou la diminution de la vitesse du ou des moteurs assurant la rotation des tambours d'étirage. Afin d'éviter des alternances ininterrompues d'accélération et de décélération des constantes de temps peuvent être introduites dans le circuit de commande, les augmentations et les diminutions de vitesse du ou des moteurs étant précisément réglées. On constitue ainsi une véritable boucle de régulation de la tension de la nappe.

La description détaillée ci-après permettra de mieux comprendre les avantages de la présente invention. Cette description est illustrée par des figures selon lesquelles :
- **la figure 1** représente une vue schématique d'un dispositif de détection de la nappe des filaments thermoplastiques et du circuit de commande de leur vitesse d'étirage dans le cadre d'une installation de fabrication d'un fil composite,
- **les figures 2A, 2B, 2C** représentent schématiquement des vues de face et de côté d'un premier mode de réalisation du dispositif d'étirage de la nappe de filaments thermoplastiques,
- **les figures 3A et 3B** représentent schématiquement un deuxième mode de réalisation du dispositif d'étirage de la nappe de filaments thermoplastiques,
- **les figures 4A, 4B et 4C** représentent schématiquement un troisième mode de réalisation du dispositif d'étirage de la nappe de filaments thermoplastiques.

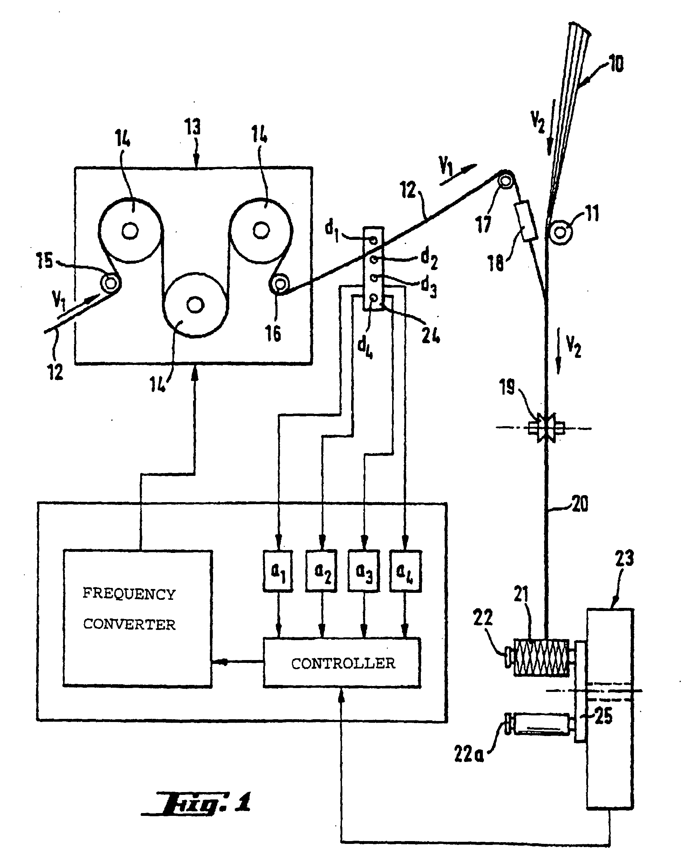

Le dispositif de détection représenté figure 1 est implanté dans une installation de fabrication d'un fil composite formé par l'association de filaments de verre et de filaments thermoplastiques continus.

Cette installation comprend une filière alimentée en verre, dont la face inférieure est munie d'une multiplicité d'orifices à partir desquels sont étirés une multiplicité de filaments continus sous la forme d'au moins un faisceau 10. Ce faisceau passe sur un dispositif enducteur, symbolisé par le rouleau 11, alimenté en apprêt ou en ensimage. A son contact, les filaments dudit faisceau de la nappe 10 se recouvrent de cet apprêt ou de cet ensimage.

Cette installation comprend également une tête de filage, non représentée, d'où sont extrudés une multiplicité de filaments continus d'une matière organique thermoplastique, telle que du polypropylène. Ces filaments sont étirés sous la forme d'une nappe 12 à l'aide d'un dispositif 13 équipé de trois tambours moteurs 14, précédés et suivis des rouleaux de renvoi 15 et 16 qui permettent d'augmenter l'arc de contact de ladite nappe avec lesdits tambours. La nappe 12 passe ensuite sur un rouleau de guidage 17 avant de traverser un dispositif venturi 18 qui la projette dans le faisceau 10 des filaments de verre ensimés.

L'ensemble des filaments du faisceau 10 et de la nappe 12 ainsi mêlés sont réunis par une roulette d'assemblage 19 en un fil composite 20.

Ce fil 20 est bobiné, par exemple sous la forme d'un enroulement à flancs droits 21, sur la broche 22 d'un bobinoir à barillet 23. Les filaments de la nappe 12 sont étirés par les tambours 14, par exemple, à une vitesse V₁ qui est supérieure à la vitesse V₂ des filaments du faisceau 10 qui sont étirés par la broche 22. Comme cela a été exposé précédemment cet écart de vitesse permet de compenser le retrait des filaments thermoplastique qui intervient avant le bobinage du fil composite 20 sur la broche 22. Cet écart de vitesse est différent selon la nature de la matière organique thermoplastique utilisée et/ou les conditions d'étirage choisies.

Le venturi 18 projette la nappe 12 à une vitesse qui est normalement identique à V₁. Il en résulte que la tension de la nappe 12, entre les derniers organes mécaniques du dispositif 13 et le venturi 18, est très faible et pratiquement constante. Cette tension est si faible que la nappe 12 forme une chaînette sous l'action de son propre poids, entre le rouleau 16 et le rouleau 17. Aucun dispositif de contrôle de tension classique n'est adapté pour mesurer un niveau de tension aussi faible.

Le dispositif selon l'invention comprend, dans le mode de réalisation illustré par la figure 1, quatre détecteurs d₁, d₂, d₃ et d₄, disposés à des hauteurs différentes qui délimitent une zone dans laquelle la nappe 12 peut se déplacer. Ces détecteurs sont fixés sur un support 24 placé entre les rouleaux 16 et 17. Chacun de ces détecteurs comprend un émetteur et un récepteur qui fonctionnent ensemble. Chaque émetteur émet un faisceau lumineux par pulsations. Lorsque ce faisceau rencontre les filaments de la nappe 12, une partie de la lumière est recueillie par le récepteur associé. L'information reçue par chaque récepteur est transformée séparément par les amplificateurs a₁, a₂, a₃, a₄ qui transmettent un signal binaire à un automate. Cet automate possède une sortie de commande (analogique ou digitale) qui est reliée à l'entrée de consigne extérieure de vitesse d'un convertisseur de fréquence pour la commande des moteurs actionnant les tambours 14. L'automate analyse en permanence l'état logique des détecteurs et grâce à un programme, règle la vitesse de rotation des tambours 14 afin de maintenir la tension de la nappe 12 à une valeur minimale comme le montre l'exemple suivant : lorsque la nappe 12 est tendue, seul le détecteur d₁ décèle sa présence ; l'automate enregistre cette situation et commande l'accélération des moteurs. La nappe 12 arrive alors au niveau de d₂ ; l'automate commande une accélération plus faible que la précédente. La nappe arrive au niveau de d₃ ; l'automate commande une stabilisation de la vitesse des moteurs. Si la nappe 12 se détend encore, le détecteur d₄ enregistre sa présence ; l'automate commande une décélération des moteurs, ce qui a pour effet de prendre un léger retard sur la vitesse de bobinage du fil 20 et de retendre la nappe 12. Afin d'éviter que les accélérations soient continûment suivies de décélération et réciproquement, le programme régissant l'automate peut comprendre des constantes de temps et les accélérations et décélérations peuvent être quantifiées.

Le dispositif selon l'invention est mis en oeuvre de préférence avec un bobinoir comprenant plusieurs broches, du type bobinoir à barillet 23. Cela permet de réaliser des enroulements successifs sans interrompre les opérations d'étirage des filaments de verre et des filaments organiques thermoplastiques.

L'opération de transfert est effectuée d'une manière connue en soi : lorsque l'enroulement 21 atteint la dimension voulue, le fil 20 est écarté vers l'extrémité libre de la broche 22 sur laquelle il s'enroule, la broche 22a étant mise en rotation, le barillet 25 opère une demie rotation de manière que ladite broche prenne la place de la broche 22. Le fil 20 s'enroule alors sur l'extrémité libre de la broche 22a avant de reprendre son trajet initial pour réaliser un nouvel enroulement.

Ce transfert s'accompagne de variations brutales de la vitesse de la nappe 12, préjudiciables à la bonne marche de l'installation.

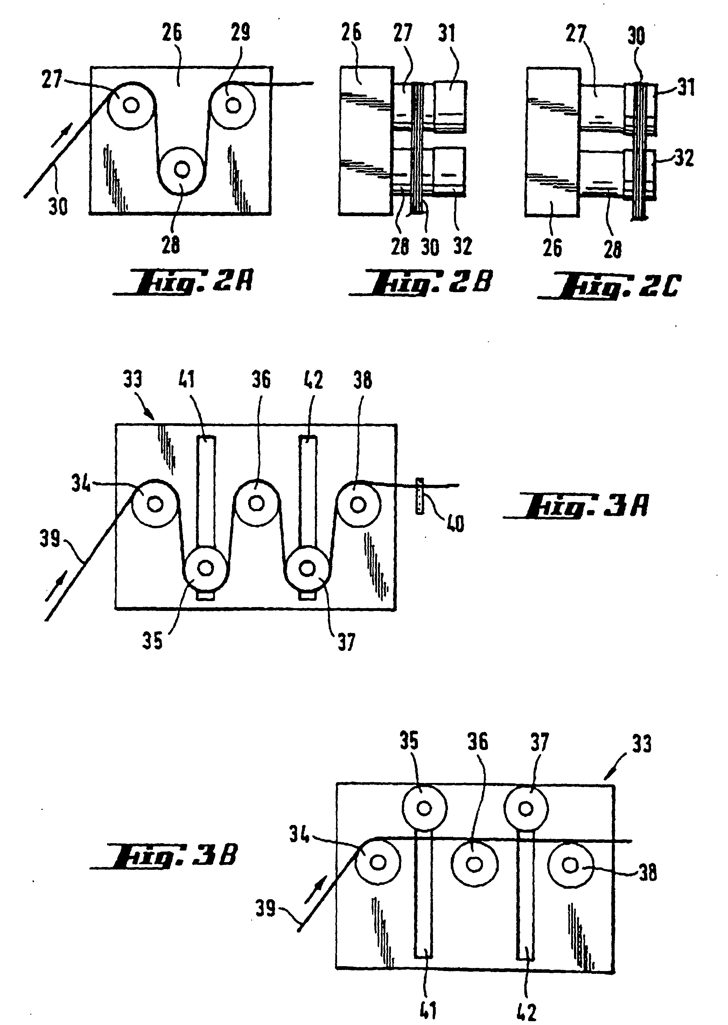

Un premier mode de réalisation, illustré par les figures 2A, 2B, 2C, permet de réduire considérablement ces variations.

La figure 2A montre un dispositif d'étirage 26 muni de trois tambours-moteurs 27, 28 et 29 entraînant la nappe de filaments thermoplastiques 30.

La figure 2B montre le dispositif d'étirage 26 ainsi que les tambours 27 et 28 sur lesquels la nappe de filaments 30 est en position d'étirage. Ces tambours sont munis à leur extrémité libre de cylindres fous 31 et 32. Le tambour 29, non représenté, est équipé également d'un cylindre fou.

Dès le début de l'opération de transfert, la nappe 30 est mise en contact avec les cylindres fous, comme le représente la figure 2C. Cette opération peut résulter soit du déplacement latéral de la nappe 30 au moyen d'un dispositif non représenté, soit du déplacement latéral de l'ensemble du dispositif 26 monté sur des glissières non représentées. La nappe 30 est alors uniquement entraînée par les filaments 10 en cours d'étirage.

L'automate, relié par un circuit au bobinoir 23, reçoit l'information de début et de fin de l'opération de transfert. Le signal du début de l'opération a pour effet de mettre hors-circuit les détecteurs de présence de la nappe et de donner aux moteurs commandant les tambours une consigne de vitesse de rotation inférieure à la vitesse d'étirage normale. Le signal de la fin de l'opération de transfert a pour effet de réactiver les détecteurs et d'augmenter la vitesse de rotation des tambours jusqu'à la vitesse d'étirage normale.

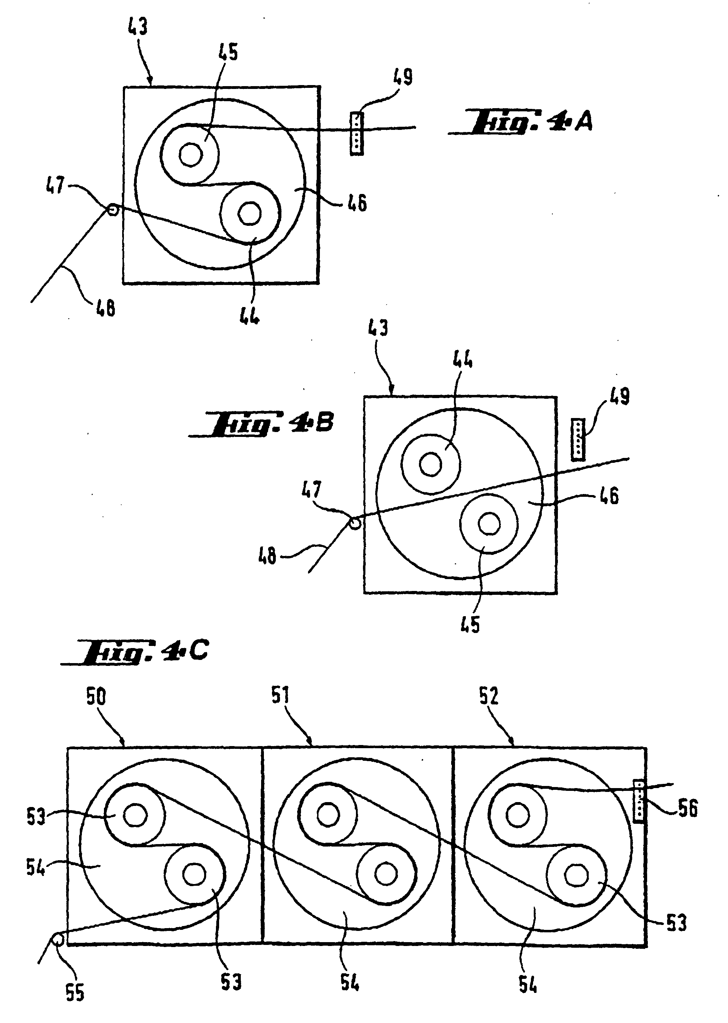

Un deuxième mode de réalisation du dispositif selon l'invention permet également de réduire les variations brutales de vitesse lors de l'opération de transfert. Ce deuxième mode de réalisation est illustré par les figures 3A et 3B.

La figure 3A représente un dispositif d'étirage 33 muni de cinq tambours-moteurs 34, 35, 36, 37 et 38 entraînant la nappe de filaments thermoplastiques 39. Dans ce dispositif, en état de marche, les tambours 34, 36 et 38 en position haute sont disposés en alternance avec les tambours 35 et 37 en position basse, le dispositif de détection 40 de la nappe étant en fonctionnement.

La figure 3B montre le même dispositif pendant l'opération de transfert.

Le signal du début de l'opération transmise à l'automate a pour effet de commander la translation verticale des tambours 35 et 37 le long des glissières 41 et 42. En fin de translation, la nappe 39 est libérée et touche uniquement la partie supérieure des tambours 34, 36 et 38. Le signal de la fin de l'opération de transfert a pour effet de commander le retour des tambours à leur position initiale. Comme précédemment ces signaux, ont pour effet de mettre hors-circuit puis de réactiver le dispositif de détection 40 et d'imposer aux moteurs commandant les tambours différentes vitesses de rotation.

Un troisième mode de réalisation du dispositif selon l'invention permet de réduire les variations brutales de vitesse lors de l'opération de transfert. Ce troisième mode de réalisation est illustré par les figures 4A, 4B et 4C.

La figure 4A représente un dispositif d'étirage 43 muni de deux tambours-moteurs 44 et 45, montés sur un barillet 46. Cette figure représente ce dispositif en position d'étirage normal. Un rouleau de guidage 47 est placé en amont du dispositif 43 de manière à augmenter l'arc de contact de la nappe de filaments 48 avec le tambour 44. En aval, le dispositif de détection 49 est en fonctionnement.

La figure 4B représente le dispositif 43 pendant l'opération de transfert.

Le signal de début de l'opération transmise à l'automate a pour effet de commander la rotation du barillet 46 de manière à libérer complètement la nappe 48. Le signal de la fin de l'opération de transfert a pour effet de commander la rotation du barillet 46 en sens inverse de manière à rétablir les tambours 44 et 45 dans leur position initiale.

Dans une variante, il est possible d'imposer au barillet 46 une rotation plus limitée de la nappe avec les tambours 44 et 45. Cette variante à l'avantage de réduire la tension imposée à la nappe 48 et permet d'utiliser une série de dispositifs d'étirage tels que représentés figure 4C.

L'installation représentée par cette figure comporte trois dispositifs 50, 51 et 52 montés en série. Chacun de ces dispositifs comprend deux tambours-moteurs 53, chaque paire de tambours étant montée sur un barillet 54. La figure 4C montre l'installation en position d'étirage normal, avec en amont du dispositif 50 un rouleau de guidage 55 et, en aval du dispositif 53, un dispositif de détection 56.

Le fonctionnement des dispositifs précédemment décrit à l'occasion d'une opération de transfert du fil composite d'une broche sur une autre, est le même lorsqu'il s'agit d'amorcer l'étirage des filaments organiques ou de la relance de leur étirage interrompu pour une raison ou pour une autre.

Un ou plusieurs des tambours des dispositifs d'étirage peuvent être munis d'éléments chauffants susceptibles de porter leur surface à une température déterminée, ces éléments sont susceptibles d'être commandés individuellement, afin de pouvoir régler séparément la température à laquelle la surface de chaque tambour sera portée. Le traitement thermique subi par la nappe de filaments lors de son contact avec la surface desdits tambours permet de stabiliser, au moins en partie, la modification structurale du polymère intervenue lors de son étirage sous la forme de filaments. Dans ce mode de réalisation, la vitesse d'étirage conférée aux filaments organiques thermoplastiques peut être à peine supérieure voire égale à celle des filaments de verre auxquels ils sont associés.

Bien que le phénomène de retrait apparaissant sur les filaments organiques thermoplastiques soit réduit, voire même supprimé du fait de ce traitement thermique, le dispositif de détection de la nappe conserve tout son intérêt puisque sa fonction essentielle est de maintenir sa tension à la valeur la plus faible et la plus constante possible, quelle que soit la vitesse d'étirage de ladite nappe.

## Revendications

1. Dispositif pour la fabrication d'un fil composite (20) formé par l'association de filaments continus de verre (10) et de filaments continus de matière organique thermoplastique (12) comprenant d'une part au moins une filière, alimentée en verre, dont la face inférieure est munie d'une multiplicité d'orifices à partir desquels sont étirés une multiplicité de filaments de verre, associée à un dispositif enducteur (11), d'autre part au moins une tête de filage alimentée en matière organique thermoplastique fondue, dont la face inférieure est munie d'une multiplicité d'orifices à partir desquels sont étirés une multiplicité de filaments organiques (12), ladite tête étant associée à un dispositif d'étirage (13, 26, 33, 43) comprenant au moins deux tambours (14, 27, 28, 29, 34, 35, 36, 37, 38, 44, 45) et à un dispositif venturi (18) projetant les filaments organiques dans la nappe formée par les filaments de verre, et des moyens, communs à la filière et à la tête de filage, permettant l'assemblage et rétirage du fil composite, par exemple par un dispositif de bobinage tel qu'un bobinoir (23) muni de broches (22), **caractérisé en ce qu'**un dispositif optique de détection (49, 56) de la nappe de filaments thermoplastiques défilant dans une zone déterminée est disposé entre le dispositif d'étirage à tambour (13, 26, 33, 43) et le dispositif venturi (18), ledit dispositif optique (49, 56) comprenant au moins 2 détecteurs (d₁, d₂, d₃, d₄) placés sur un support (24) à des hauteurs différentes qui définissent les limites d'une zone dans laquelle ladite nappe peut se déplacer pendant l'opération d'étirage des filaments thermoplastiques et étant relié à un circuit commandant la vitesse de rotation dont au moins un des tambours, en réponse à l'état logique des détecteurs.

2. Dispositif selon la revendication 1, **caractérisé en ce que** les détecteurs (d₁, d₂, d₃, d₄) sont disposés d'un seul côté de la nappe et sont munis chacun d'un émetteur, susceptible d'émettre un faisceau lumineux, et d'un récepteur susceptible de recevoir au moins une partie de la lumière réfléchie par les filaments thermoplastiques rencontrant ledit faisceau.

3. Dispositif selon la revendication 1, **caractérisé en ce que** les détecteurs (d₁, d₂, d₃, d₄) sont constitués d'émetteurs et de récepteurs disposés de part et d'autre de la nappe de filaments thermoplastiques et coopérant par paire.

4. Dispositif selon l'une quelconque des revendications 2 et 3, **caractérisé en ce que** les émetteurs émettent un faisceau lumineux par pulsations régulières.

5. Dispositif selon l'une quelconque des revendications 2 à 4, **caractérisé en ce que** les récepteurs des détecteurs (d₁, d₂, d₃, d₄) sont reliés, via un amplificateur (a₁, a₂, a₃, a₄), à un automate dont la sortie est reliée au dispositif de commande du ou des moteurs commandant la vitesse de rotation des tambours (14, 27, 28, 29, 34, 35, 36, 37, 38, 44, 45).

6. Dispositif selon la revendication 5, **caractérisé en ce que** l'automate est relié au dispositif de bobinage (23) par un circuit qui lui transmet l'information de début et de fin de l'opération de transfert du fil composite (20) ou de l'opération de relance de l'étirage des filaments thermoplastiques.

7. Dispositif selon la revendication 6, **caractérisé en ce que** l'automate est régi par un programme qui commande la mise hors circuit du dispositif de détection, la variation de la vitesse de rotation des tambours (14, 27, 28, 29, 34, 35, 36, 37, 38, 44, 45) et le déplacement éventuel desdits tambours au début et à la fin de l'opération de transfert du fil composite (20) ou de l'opération de relance de l'étirage des filaments thermoplastiques.

8. Dispositif selon la revendication 7, **caractérisé en ce que** l'extrémité libre des tambours (14, 27, 28, 29, 34, 35, 36, 37, 38, 44, 45) du dispositif d'étirage (13, 26, 33, 43) comporte un cylindre fou (31, 32) monté sur l'axe de chacun desdits tambours.

9. Dispositif selon la revendication 8, **caractérisé en ce que** le dispositif d'étirage à tambours (13, 26, 33, 43) est mobile et susceptible de s'écarter et de se rapprocher latéralement du trajet suivi par la nappe de filaments thermoplastiques (12).

10. Dispositif selon la revendication 9, **caractérisé en ce que**, lors de l'opération de transfert du fil composite ou lors de l'opération de relance de l'étirage des filaments thermoplastiques, ledit dispositif s'écarte jusqu'à ce que la nappe de filaments thermoplastiques (12) vienne en contact avec les cylindres fous (31, 32) puis reprend sa position initiale à l'issue de ladite relance ou dudit transfert.

11. Dispositif selon la revendication 7, **caractérisé en ce que** les tambours (14, 27, 28, 29, 34, 35, 36, 37, 38, 44, 45) du dispositif d'étirage (13, 26, 33, 43) sont montés sur au moins un barillet (46) susceptible de tourner autour d'un axe parallèle à l'axe de rotation desdits tambours.

12. Dispositif selon la revendication 11, **caractérisé en ce que**, lors de l'opération de transfert ou de relance, le barillet (46) tourne afin d'écarter les tambours (14, 27, 28, 29, 34, 35, 36, 37, 38, 44, 45) de la nappe de filaments thermoplastiques (12) au début de ladite opération, reprenant sa position initiale à l'issue de ladite opération.

13. Dispositif selon la revendication 7, **caractérisé en ce que** les tambours (14, 27, 28, 29, 34, 35, 36, 37, 38, 44, 45) du dispositif d'étirage (13, 26, 33, 43) sont montés sur un bâti fixe, l'un au moins des tambours étant susceptible de se déplacer perpendiculairement à la nappe de filaments thermoplastiques.

14. Dispositif selon la revendication 13, **caractérisé en ce que**, lors de l'opération de transfert ou de relance, au moins un des tambours (14, 27, 28, 29, 34, 35, 36, 37, 38, 44, 45) s'écarte de la nappe de filaments thermoplastiques (12) au début de l'opération puis revient à sa position initiale à l'issue de ladite opération.

15. Dispositif selon l'une quelconque des revendications 8, 11 et 13,
**caractérisé en ce qu'**un ou plusieurs des tambours (14, 27, 28, 29, 34, 35, 36, 37, 38, 44, 45) du dispositif d'étirage (13, 26, 33, 43) sont susceptibles d'être chauffés afin de stabiliser la structure des filaments thermoplastiques.

16. Dispositif selon la revendication 15, **caractérisé en ce que** le dispositif d'étirage (13, 26, 33, 43) comprend plusieurs tambours (14, 27, 28, 29, 34, 35, 36, 37, 38, 44, 45) munis d'un moyen de chauffage, chacun des moyens étant susceptible d'être commandé individuellement de manière à régler séparément la température à laquelle la surface de chaque tambour sera portée.

## Patentansprüche

1. Vorrichtung zur Herstellung eines Hybridfadens (20), gebildet durch Verbindung endloser Glasfilamente (10) mit endlosen Filamenten aus thermoplastischem organischem Werkstoff (12), bestehend aus einerseits mindestens einer Spinndüse, der Glas zugeführt wird und deren Unterseite mit einer Vielzahl von Öffnungen versehen ist, aus denen eine Vielzahl von Glasfilamenten gezogen wird, verbunden mit einer Beschichtungsvorrichtung (11), andererseits mindestens einem Spinnkopf, dem geschmolzener thermoplastischer organischer Werkstoff zugeführt wird, dessen Unterseite mit einer Vielzahl von Öffnungen versehen ist, aus denen eine Vielzahl organischer Filamente (12) gezogen wird, wobei der genannte Kopf mit einer Streckvorrichtung (13, 26, 33, 43) verbunden ist, die mindestens zwei Rollen (14, 27, 28, 29, 34, 35, 36, 37, 38, 44, 45) umfasst, und mit einer Venturivorrichtung (18), die die organischen Filamente in die von den Glasfilamenten gebildete Schar schleudert, und der Spinndüse und dem Spinnkopf gemeinsame Mittel, die das Zusammensetzen und Strecken des Hybridfadens ermöglichen, beispielsweise durch eine Wickelvorrichtung, wie etwa eine mit Spindeln (22) versehene Wickelmaschine (23), **dadurch gekennzeichnet, dass** eine optische Abtastvorrichtung (49, 56) der Schar thermoplastischer Filamente, die eine festgelegte Zone durchläuft, zwischen der Rollenstreckvorrichtung (13, 26, 33, 43) und der Venturivorrichtung (19) angeordnet ist, wobei die genannte optische Vorrichtung (49. 56) mindestens 2 Abtaster (d₁, d₂, d₃, d₄) umfasst, die auf einem Träger (24) in unterschiedlichen Höhen angeordnet sind, die die Grenzen einer Zone definieren, in der sich die genannte Schar während des Streckvorganges der thermoplastischen Filamente bewegen kann, und mit einer Schaltung verbunden ist, die die Rotationsgeschwindigkeit mindestens einer der Rollen in Abhängigkeit vom logischen Zustand der Abtaster steuert.

2. Vorrichtung nach Patentanspruch 1, **dadurch gekennzeichnet, dass** die Abtaster (d₁, d₂, d₃, d₄) auf nur einer Seite der Schar angeordnet und jeweils mit einem Sender versehen sind, der in der Lage ist, einen Lichtstrahl auszusenden, und mit einem Empfänger, der in der Lage ist, mindestens einen Teil des von den thermoplastischen Filamenten, auf die der genannte Strahl trifft, reflektierten Lichtes zu empfangen.

3. Vorrichtung nach Patentanspruch 1, **dadurch gekennzeichnet, dass** die Abtaster (d₁, d₂, d₃, d₄) aus Sendern und Empfängern bestehen, die beiderseits der Schar thermoplastischer Filamente angeordnet sind und paarweise zusammenwirken.

4. Vorrichtung nach irgendeinem der Patentansprüche 2 und 3, **dadurch gekennzeichnet, dass** die Sender einen Lichtstrahl in regelmäßigen Pulsationen aussenden.

5. Vorrichtung nach irgendeinem der Patentansprüche 2 bis 4, **dadurch gekennzeichnet, dass** die Empfänger der Abtaster (d₁, d₂, d₃, d₄) über einen Verstärker (a₁, a₂, a₃, a₄) mit einem Automaten verbunden sind, dessen Ausgang mit der Steuervorrichtung des oder der Motoren verbunden ist, die die Rotationsgeschwindigkeit der Rollen (14, 27, 28, 29, 34, 35, 36, 37, 38, 44, 45) steuert.

6. Vorrichtung nach Patentanspruch 5, **dadurch gekennzeichnet, dass** der Automat mit der Wickelvorrichtung (23) über eine Schaltung verbunden ist, die ihr die Informationen über Anfang und Ende des Transportvorganges des Hybridfadens (20) oder des Vorganges Wiederbeginn des Streckens der thermoplastischen Filamente übermittelt.

7. Vorrichtung nach Patentanspruch 6, **dadurch gekennzeichnet, dass** der Automat durch ein Programm gesteuert wird, das die Ausschaltung der Abtastvorrichtung, die Änderung der Rotationsgeschwindigkeit der Rollen (14, 27, 28, 29, 34, 35, 36, 37, 38, 44, 45) und die eventuelle Verschiebung der genannten Rollen am Anfang und Ende des Transportvorganges des Hybridfadens (20) oder des Vorganges Wiederbeginn des Streckens der thermoplastischen Filamente steuert.

8. Vorrichtung nach Patentanspruch 7, **dadurch gekennzeichnet, dass** das freie Ende der Rollen (14, 27, 28, 29, 34, 35, 36, 37, 38, 44, 45) der Rollenstreckvorrichtung (13, 26, 33, 43) einen losen Zylinder (31, 32) trägt, der auf der Achse jeder der genannten Rollen angebracht ist.

9. Vorrichtung nach Patentanspruch 8, **dadurch gekennzeichnet, dass** die Rollenstreckvorrichtung (13, 26, 33, 43) beweglich ist und in der Lage, sich seitlich von der Bahn, die die Schar thermoplastischer Filamente (12) durchläuft, abzuspreizen und ihr anzunähern.

10. Vorrichtung nach Patentanspruch 9, **dadurch gekennzeichnet, dass** sich die genannte Vorrichtung während des Transportvorganges des Hybridfadens oder des Vorganges Wiederbeginn des Streckens der thermoplastischen Filamente spreizt, bis die Schar thermoplastischer Filamente (12) mit den losen Zylindern (31, 32) in Berührung kommt, und dann am Ende des genannten Wiederbeginns oder Transportes ihre Ausgangsstellung wieder einnimmt.

11. Vorrichtung nach Patentanspruch 7, **dadurch gekennzeichnet, dass** die Rollen (14, 27, 28, 29, 34, 35, 36, 37, 38, 44, 45) der Rollenstreckvorrichtung (13, 26, 33, 43) auf mindestens einer Trommel (46) montiert sind, die in der Lage ist, sich um eine Achse zu drehen, die zur Rotationsachse der genannten Rollen parallel ist.

12. Vorrichtung nach Patentanspruch 11, **dadurch gekennzeichnet, dass** sich die Trommel (46) beim Transportvorgang oder Wiederbeginnvorgang dreht, um die Rollen (14, 27, 28, 29, 34, 35, 36, 37, 38, 44, 45) von der Schar thermoplastischer Filamente (12) zu Beginn des genannten Vorganges abzuspreizen, um am Ende des genannten Vorganges ihre Ausgangsstellung wiedereinzunehmen.

13. Vorrichtung nach Patentanspruch 7, **dadurch gekennzeichnet, dass** die Rollen (14, 27, 28, 29, 34, 35, 36, 37, 38, 44, 45) der Rollenstreckvorrichtung (13, 26, 33, 43) in einem feststehenden Gehäuse montiert sind, wobei mindestens eine der Rollen in der Lage ist, sich senkrecht zur Schar thermoplastischer Filamente zu verschieben.

14. Vorrichtung nach Patentanspruch 13, **dadurch gekennzeichnet, dass** sich beim Transportvorgang oder Wiederbeginnvorgang mindestens eine der Rollen (14, 27, 28, 29, 34, 35, 36, 37, 38, 44, 45) von der Schar thermoplastischer Filamente (12) zu Beginn des genannten Vorganges abspreizt, um am Ende des genannten Vorganges ihre Ausgangsstellung wiedereinzunehmen.

15. Vorrichtung nach irgendeinem der Patentansprüche 8, 11 und 13, **dadurch gekennzeichnet, dass** eine oder mehrere der Rollen (14, 27, 28, 29, 34, 35, 36, 37, 38, 44, 45) der Rollenstreckvorrichtung (13, 26, 33, 43) beheizt werden können, um die Struktur der thermoplastischen Filamente zu stabilisieren.

16. Vorrichtung nach Patentanspruch 15, **dadurch gekennzeichnet, dass** die Rollenstreckvorrichtung (13, 26, 33, 43) mehrere Rollen (14, 27, 28, 29, 34, 35, 36, 37, 38, 44, 45) enthält, die mit einem Heizmittel versehen sind, wobei jedes der Mittel einzeln derart gesteuert werden kann, dass getrennt die Temperatur geregelt wird, auf die die Oberfläche jeder Rolle gebracht wird.

## Claims

1. Device for production of a composite fibre (20) formed by the association of continuous filaments of glass (10) and continuous filaments of thermoplastic organic material (12), comprising firstly at least one die supplied with glass, the lower face of which is fitted with a multiplicity of openings through which are drawn a multiplicity of glass filaments, and which die is associated with the coating device (11), secondly at least one extrusion head supplied with molten thermoplastic organic material, the lower face of which is fitted with a multiplicity of openings from which are drawn a multiplicity of organic filaments (12), said head being associated with a drawing device (13, 26, 33, 43) comprising at least two drums (14, 27, 28, 29, 34, 35, 36, 37, 38, 44, 45) and a Venturi device (18) projecting the organic filaments into the sheet formed by the glass filaments, and means common to the die and the extrusion head allowing assembly and drawing of the composite fibre, for example via a winding device such as a winder (23) fitted with pins (22), **characterised in that** an optical detection device (49, 56) for the sheet of thermoplastic filaments running off into a particular zone is arranged between the drum drawing device (13, 26, 33, 43) and the Venturi device (18), said optical device (49, 56) comprising at least two detectors (d₁, d₂, d₃, d₄) placed on a support (24) at different heights which define the limits of a zone within which the said sheet can move during the drawing operation of the thermoplastic filaments, and being linked to a circuit controlling the rotation speed of at least one of the drums in response to the logic state of the detectors.

2. Device according to claim 1, **characterised in that** the detectors (d₁, d₂, d₃, d₄) are arranged on a single side of the sheet and are each fitted with an emitter able to emit a light beam and a receiver able to receive at least part of the light reflected by the thermoplastic filaments encountering said beam.

3. Device according to claim 1, **characterised in that** the detectors (d₁, d₂, d₃, d₄) comprise emitters and receivers arranged on either side of the sheet of thermoplastic filaments and co-operating in pairs.

4. Device according to either claim 2 or claim 3, **characterised in that** the emitters emit a light beam by regular pulses.

5. Device according to any one of claims 2 to 4, **characterised in that** the receivers of the detectors (d₁, d₂, d₃, d₄) are connected via an amplifier (a₁, a₂, a₃, a₄) to an automatic device, the output of which is linked to the control device of the motor or motors controlling the rotation speed of the drums (14, 27, 28, 29, 34, 35, 36, 37, 38, 44, 45).

6. Device according to claim 5, **characterised in that** the automatic device is connected to the winding device (23) by a circuit which gives it the information on the start and the end of the operation of transferring the composite fibre (20) or the operation of restarting the drawing of the thermoplastic filaments.

7. Device according to claim 6, **characterised in that** the automatic device is controlled by a program which controls the disconnection of the detection device, the variation in rotation speed of the drums (14, 27, 28, 29, 34, 35, 36, 37, 38, 44, 45) and any movement of the said drums at the start and end of the operation of transferring the composite fibre (20) or the operation of restarting the drawing of the thermoplastic filaments.

8. Device according to claim 7, **characterised in that** the free end of the drums (14, 27, 28, 29, 34, 35, 36, 37, 38, 44, 45) of the drawing device (13, 26, 33, 43) comprises an idle cylinder (31, 32) mounted on the axle of each of the said drums.

9. Device according to claim 8, **characterised in that** the drum drawing device (13, 26, 33, 43) is mobile and able to move laterally away from and towards the path followed by the sheet of thermoplastic filaments (12).

10. Device according to claim 9, **characterised in that** during the transfer of the composite fibre or during the restarting of the drawing of the thermoplastic filaments, the said device moves away until the sheet of thermoplastic filaments (12) comes into contact with the idle cylinders (31, 32) and then resumes its initial position at the end of the said restart or transfer operation.

11. Device according to claim 7, **characterised in that** the drums (14, 27, 28, 29, 34, 35, 36, 37, 38, 44, 45) of the drawing device (13, 26, 33, 43) are mounted on at least one barrel (46) able to rotate about an axis parallel to the axis of rotation of the said drums.

12. Device according to claim 11, **characterised in that** during the transfer or restart operation, the barrel (46) rotates in order to move the drums (14, 27, 28, 29, 34, 35, 36, 37, 38, 44, 45) away from the sheet of thermoplastic filaments (12) at the start of the said operation, resuming its initial position at the end of said operation.

13. Device according to claim 7, **characterised in that** the drums (14, 27, 28, 29, 34, 35, 36, 37, 38, 44, 45) of the drawing device (13, 26, 33, 43) are mounted on a fixed frame, at least one of the drums being able to move perpendicular to the sheet of thermoplastic filaments.

14. Device according to claim 13, **characterised in that** during the transfer or start operation at least one of the drums (14, 27, 28, 29, 34, 35, 36, 37, 38, 44, 45) moves away from the sheet of thermoplastic filaments (12) at the start of the operation then resumes its initial position at the end of the said operation.

15. Device according to any one of claims 8, 11 and 13, **characterised in that** one or more of the drums (14, 27, 28, 29, 34, 35, 36, 37, 38, 44, 45) of the drawing device (13, 26, 33, 43) is/are able to be heated in order to stabilise the structure of the thermoplastic filaments.

16. Device according to claim 15, **characterised in that** the drawing device (13, 26, 33, 43) comprises several drums (14, 27, 28, 29, 34, 35, 36, 37, 38, 44, 45) fitted with heating means, each of the means being able to be controlled individually so as to adjust separately the temperature to which the surface of each drum will be brought.
